# EUROPEAN PATENT APPLICATION

(11) **EP 4 678 742 A1**
(43) Date of publication of application: **14.01.2026**
(21) Application number: 24187793.5
(22) Date of filing: 10.07.2024
(51) Int. Cl.: C12N 9/22, C12N 15/52, C12N 15/62

(54) **IMPROVED CAS9 PROTEINS**

(71) Applicant: Astrazeneca AB, 151 85 Södertälje (SE)
(72) Inventor: ZDRAVKOVI , Aleksandar, SÖDERTÄLJE (SE); SIENSKI, Grzegorz, SÖDERTÄLJE (SE); MARESCA, Marcello, SÖDERTÄLJE (SE)
(74) Representative: Mewburn Ellis LLP

(57) **Abstract**

The disclosure relates to a Cas9 protein comprising a polypeptide sequence having at least 95% identity, such as 97%, 98% or 99%, to SEQ ID NO: 1, wherein said polypeptide sequence comprises an amino acid modification at one or more positions relative to SEQ ID NO: 1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229, CRISPR-Cas systems and methods for providing site-specific modification of a target sequence in a eukaryotic cell.

## Description

### FIELD OF THE INVENTION

The present disclosure generally relates to improved Cas9 proteins, CRISPR-Cas9 systems and methods for providing site-specific modification of a target sequence in a eukaryotic cell using improved Cas9 proteins.

### BACKGROUND

Genome editing has undergone significant evolution, with systems like zinc fingers and TALENs showing limitations in precision and ease of use. In contrast, CRISPR-Cas9 systems offer greater ease of design and implementation. The CRISPR-Cas9 systems originate as a natural bacterial immune system, where they function as a defence mechanism against viral infections. These systems use guide RNA to target specific DNA sequences with a nuclease protein (Cas9), making them versatile and easy to implement across various applications, including genome editing.

The CRISPR-Cas9 gene editing system has been used successfully in a wide range of organisms and cell lines, both in order to induce double stranded break (DSB) formation in DNA using the wild type Cas9 protein or to nick a single DNA strand using a mutant protein termed Cas9n/Cas9 D10A (*see*, *e.g.*, Mali et al., Science, 339 (6121): 823-826 (2013) and Sander and Joung, Nature Biotechnology 32(4): 347-355 (2014), each of which is incorporated by reference herein in its entirety).

In addition to genome editing, the CRISPR system has a multitude of other applications, including regulating gene expression, genetic circuit construction, and functional genomics, amongst others (reviewed in Sander and Joung, 2014).

While some Cas9 proteins have been shown to be effective in a wide variety of *in vivo* and *in vitro* applications, their relatively large size is not suitable for fitting into delivery methods (e.g., AVV) for therapeutic applications.

Cas9 proteins have been isolated from different bacteria, including *S. aureus* and S. *thermophilus.* Cas9 proteins from *Campylobacter lanienae* have been previously described by WO2019099943A1 (which is incorporated by reference herein in its entirety), however the wild-type enzyme displays a very low activity, which is incompatible with genome editing for therapeutic applications.

There is a need for a highly effective Cas9 enzyme, which has also potentially a compatible size to be packaged into delivery methods, for instance AAV, and thus which is suitable for genome editing therapy in humans.

### SUMMARY OF THE INVENTION

The present disclosure is directed to a Cas9 protein comprising a polypeptide sequence having at least 95% identity, such as 97%, 98% or 99%, to SEQ ID NO: 1, wherein said polypeptide sequence comprises an amino acid modification at one or more positions relative to SEQ ID NO:1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229. In one embodiment, said polypeptide sequence consists of an amino acid modification at one or more positions relative to SEQ ID NO:1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229.

According to one embodiment, said Cas9 protein is an engineered and/or a non-naturally occurring Cas9 protein.

According to another embodiment, the amino acid modification is an amino acid substitution at one or more positions relative to SEQ ID NO:1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229.

According to one embodiment, said Cas9 protein comprises an enhanced endonuclease activity compared to a wild type Cas9 protein, such as a wild type Cl1Cas9 protein, optionally wherein said endonuclease activity is measured by using the Xential method as per Li S et al., Nat Commun 12, 497 (2021) (which is incorporated by reference herein in its entirety) on wild type HEK293T cells (see, for instance, Example 1 and/or Example 2).

According to one embodiment, said Cas9 protein comprises an enhanced endonuclease activity and/or an enhanced fidelity compared to SEQ ID NO 208, optionally wherein said endonuclease activity and/or fidelity is optionally measured by using the Xential method as per Li S et al., Nat Commun 12, 497 (2021) (which is incorporated by reference herein in its entirety) on wild type HEK293T cells (see, for instance, Example 1 and/or Example 2).

According to one embodiment, the polypeptide sequence comprises or consists of one or more amino acid modifications selected from D1073A, A1160R, D1073G, D1091G, D1091N, D1091R, D1091S, D1122R, D1206G, D1206N, D1206R, D1206S, D37G, D37N, D37R, D47G, D47L, D47R, D59G, D59R, D59S, D61G, D61L, D61R, D911G, D911K, D911R, D911S, D912G, D912N, D912R, D912S, E1164R, E1226G, E1226R, E1226S, E508G, E508R, F94G, F94Y, G39R, I46A, I46R, I978R, K980R, N1161R, N501F, N501R, N501W, N501Y, Q315K, Q315R, Q556R, S1229R, S566K, S566R, S57A, S57L, S57R, and T54R.

According to one embodiment, the polypeptide sequence comprises a first amino acid modification selected from D37R, D1091R, D912R, G39R, I978R, N1161R, and S1229R.

According to one embodiment, wherein the polypeptide sequence further comprises a second amino acid modification selected from D1091R, D912R, G39R, N1161R, and S1229R, optionally wherein the first amino acid modification is D37R and the second amino acid modification is selected from D1091R, N1161R, D912R, S1229R, and G39R, optionally wherein the first amino acid modification is D1091R and the second amino acid modification is selected from N1161R, S1229R, and D912R.

According to one embodiment, the Cas9 protein further comprises a third amino acid modification selected from A1160R, D1073A, D1073G, D1091G, D1091N, D1091R, D1091S, D1122R, D1206G, D1206R, D1206S, D37G, D37N, D47G, D47L, D47R, D59G, D59R, D59S, D61G, D61L, D61R, D911G, D911S, D912G, D912N, D912R, D912S, E1164R, E1226G, E1226R, E1226S, E508G, E508R, F94G, F94Y, I46A, I46R, I978R, K980R, N1161R, N501F, N501R, N501W, N501Y, Q315K, Q315R, S1229R, S566K, S566R, S57A, S57L, S57R, and T54R.

According to one embodiment, the Cas9 protein further comprises a fourth amino acid modification selected from D1206S, D59S, D912N, D912R, D912S, E1164R, E1226G, E1226S, F94Y, I46R, Q556R, S1229R, S566R, and S57R.

According to one embodiment, the Cas9 protein further comprises a fifth amino acid modification selected from D1206N, D1206S, D912N, D912R, D912S, G39R, I46R, and S1229R, optionally a sixth amino acid modification selected from S1229R, S566R, and optionally a seventh amino acid modification at position D912N.

According to one embodiment, said Cas9 protein comprises one or more mutations as defined in Table 2, or wherein said Cas9 protein comprises or consists of an amino acid sequence as defined in any one of SEQ ID NOs: 9-191, such as SEQ ID NOs: 9-144, SEQ ID NOs: 14-144, SEQ ID NOs: 30-144, SEQ ID NOs: 60-144, SEQ ID NOs: 112-144 and/or SEQ ID NOs: 140-144.

According to another embodiment, said Cas9 protein further comprises at least one nuclear localization signal (NLS), such as a first nuclear localization signal attached to the N-terminus of the Cas9 protein and/ or a second nuclear localization signal attached to the C-terminus of the Cas9 protein. In some embodiments, said at least one nuclear localization signal (NLS) is as defined in Table 3, such as VC, 53BP1, bpSV40, BRCA1_1, BRCA1 2, EWS, FUS, HIV-I TAT, hnRNP-D, hnRNP-M, HsNPM2, HTLV-1, mpSV40, MYC, NLP, NPM2, PTHrP and/or REV_HV1H2.

In some embodiments, said Cas9 protein further comprises at least one nuclear localization signal (NLS) attached to the N-terminus of the Cas9 protein, such as 53BP1, bpSV40, BRCA1_1, BRCA1 2, FUS, HIV-I TAT, hnRNP-D, HsNPM2, HTLV-1, mpSV40, MYC, NLP, NPM2, PTHrP, and/or REV_HV1H2.

In some embodiments, said Cas9 protein further comprises at least one nuclear localization signal (NLS) attached to the C-terminus of the Cas9 protein, such as 53bp1, 53BP1, bpSV40, BRCA1_1, BRCA1 2, EWS, FUS, HIV-I TAT, hnRNP-D, hnRNP-M, HsNPM2, HTLV-1, mpSV40, MYC, NLP, PTHrP and/or REV_HV1H2.

In some embodiments, said Cas9 protein further comprises at least one nuclear localization signal (NLS) attached to the N-terminus of the Cas9 protein, such as bpSV40, NLP, or NPM2, and said Cas9 protein further comprises at least one nuclear localization signal (NLS) attached to the C-terminus of the Cas9 protein, such as NLP, bpSV40, 53bp1, MYC, or mpSV40.

Any one or more NLS as defined in Table 3 may be combined with any one of the Cas9 proteins of SEQ ID NO: 9-191, such as SEQ ID NOs: 9-144, SEQ ID NOs: 14-144, SEQ ID NOs: 30-144, SEQ ID NOs: 60-144, SEQ ID NOs: 112-144 and/or. SEQ ID NOs: 140-144. Any one or more NLS may advantageously increase the activity of the Cas9 proteins as defined herein.

According to one embodiment, said Cas9 protein comprises or consists of an amino acid sequence as defined in any one of SEQ ID NOs: 9-191, such as SEQ ID NOs: 9-144, SEQ ID NOs: 14-144, SEQ ID NOs: 30-144, SEQ ID NOs: 60-144, SEQ ID NOs: 112-144 and/or. SEQ ID NOs: 140-144.

Another aspect of the disclosure relates to a CRISPR-Cas system comprising a Cas9 protein comprising a polypeptide sequence having at least 95% identity, such as 97%, 98% or 99%, to SEQ ID NO: 1, wherein said polypeptide sequence comprises or consists of an amino acid modification at one or more positions relative to SEQ ID NO: 1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229, or a Cas9 protein as defined elsewhere herein; and a guide polynucleotide comprising a guide sequence, wherein the guide sequence is capable of hybridizing with a target sequence in a eukaryotic cell. In some embodiments, the eukaryotic cell may be an animal or a human cell.

Another aspect of the disclosure relates to a CRISPR-Cas system comprising a nucleic acid sequence encoding a Cas9 protein comprising a polypeptide sequence having at least 95% identity, such as 97%, 98% or 99%, to SEQ ID NO: 1, wherein said polypeptide sequence comprises or consists of an amino acid modification at one or more positions relative to SEQ ID NO: 1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229, or a Cas9 protein as defined elsewhere herein; and a nucleic acid sequence encoding a guide polynucleotide that comprises a guide sequence, wherein the guide sequence is capable of hybridizing with a target sequence in a eukaryotic cell. In some embodiments, the eukaryotic cell may be an animal or a human cell.

According to one embodiment, the guide polynucleotide is an RNA and/or the nucleic acid sequence is a DNA.

According to one embodiment, the guide polynucleotide has from 80 to 140 bases in length, such as from 90 to 130 bases in length.

According to one embodiment, the guide polynucleotide comprises or consists of a polynucleotide sequence as defined in any one of SEQ ID NO: 192-206. In some embodiments, the spacer sequence comprises or consists of SEQ ID NO: 207.

Another aspect of the disclosure relates to a eukaryotic cell or a delivery particle comprising the Cas9 protein as defined elsewhere herein, or the CRISPR-Cas system as defined elsewhere herein. The delivery particle may be a vesicle or a viral vector.

According to another embodiment, the delivery particle is a vesicle or a viral vector. In some embodiments, the delivery particle is a lipid-based system, a liposome, a micelle, a microvesicle, an exosome or a gene gun. In some embodiments, the delivery particle is a lipid envelope. In some embodiments, the delivery particle is a sugar-based particle, for example, GalNAc. In some embodiments, the delivery particle is a nanoparticle. Preparation of delivery particles is further described in U.S. Patent Publication Nos. 2011/0293703, 2012/0251560, and 2013/0302401; and U.S. Patent Nos. 5,543 ,158, 5,855,913, 5,895,309, 6,007,845, and 8,709,843, each of which is incorporated herein by reference in its entirety.

Another aspect of the disclosure relates to a method for providing site-specific modification of a target sequence in a eukaryotic cell, the method comprising:
a) introducing into the eukaryotic cell a nucleotide sequence encoding a guide polynucleotide that forms a complex with a Cas9 protein , wherein said guide polynucleotide comprises a guide sequence,
   wherein the guide sequence is capable of hybridizing with a target sequence in host polynucleotide; and wherein the eukaryotic cell comprises the Cas9 protein as defined elsewhere herein;
b) generating cohesive ends in the host polynucleotide with the Cas9 protein and the guide polynucleotide; and
c) ligating
   i) the cohesive ends of (b) together, or
   ii) a 3' end of a polynucleotide sequence of interest to a first cohesive end of the host polynucleotide, and a 5' end of the polynucleotide sequence of interest to a second cohesive end of the host polynucleotide; thereby modifying the target sequence.

According to another embodiment, step a) further comprises i) introducing into the eukaryotic cell a nucleotide sequence encoding the Cas9 protein as defined elsewhere herein, and ii) expressing the Cas9 protein by the eukaryotic cell.

According to another embodiment, the guide polynucleotide is an RNA.

According to another embodiment, the guide polynucleotide has from 80 to 140 bases in length, such as from 90 to 130 bases in length.

According to one embodiment, the guide polynucleotide further comprises a tracrRNA sequence. A "tracrRNA," or trans-activating CRISPR-RNA, forms an RNA duplex with a pre-crRNA, or pre-CRISPR-RNA, and is then cleaved by the RNA specific ribonuclease RNase III to form a crRNA/tracrRNA hybrid. In some embodiments, the tracrRNA component of the guide RNA activates the Cas9 protein.

According to another embodiment, the guide polynucleotide comprises or consists of a polynucleotide sequence as defined in any one of SEQ ID NO: 192-206.

### BRIEF DESCRIPTION OF THE DRAWINGS

The following drawings form part of the present specification and are included to further demonstrate exemplary embodiments of certain aspects of the present invention.

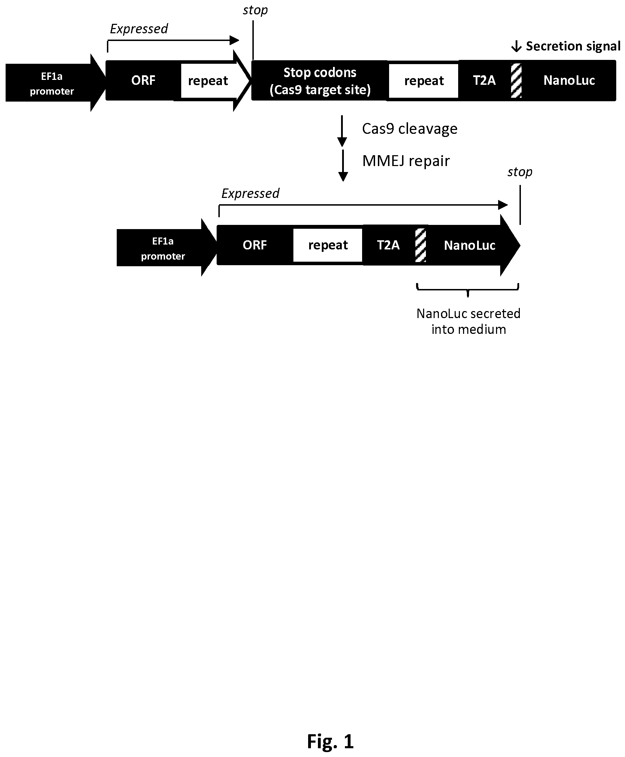

Fig. 1 provides a diagram representation of the reporter system used for comparing nucleases in terms of editing activity. Nanoluciferase release into the medium is proportional to the targeted cleavage of the described reporter cassette integrated into the HBEGF locus.
Fig. 2 - First generation of engineered Cl1pCas9 enzymes compared in terms of editing reporter activation. Representative previous generation Cl1pCas9 (namely wild type or wt) shown for reference. A) Raw luminescence signal. B) Fold change of signal (editing) compared to wtCl1Cas9.
Fig. 3 - Second generation of engineered Cl1pCas9 enzymes compared in terms of editing reporter activation. The enzymes ePsCas9 and SpCas9 are included as controls. A) Raw luminescence signal. B) Fold change of signal (editing) compared to wtCl1Cas9.
Fig. 4 - Third generation (Part 1) of engineered Cl1pCas9 enzymes compared in terms of editing reporter activation. All samples were run side-by-side with the ones presented in Figures 5 and 6, wtCl1Cas9 and previous generation Cl1pCas9 variants are shown on all figures for reference. A) Raw luminescence signal. B) Fold change of signal (editing) compared to wtCl1Cas9.
Fig. 5 - Third generation (Part 2) of engineered Cl1pCas9 enzymes compared in terms of editing reporter activation. All samples were run side-by-side with the ones presented in Figures 4 and 6, wtCl1Cas9 and previous generation Cl1pCas9 variants are shown on all figures for reference. A) Raw luminescence signal. B) Fold change of signal (editing) compared to wtCl1Cas9.
Fig 6. Third generation (Part 3) of engineered Cl1pCas9 enzymes compared in terms of editing reporter activation. All samples were run side-by-side with the ones presented in Figures 4 and 5, wtCll Cas9 and previous generation Cl1pCas9 variants are shown on all figures for reference. A) Raw luminescence signal. B) Fold change of signal (editing) compared to wtCl1Cas9.
Fig. 7 - Fourth generation of engineered Cl1pCas9 enzymes compared in terms of editing reporter activation. Representative previous generation Cl1pCas9 variants shown for reference. A) Raw luminescence signal. B) Fold change of signal (editing) compared to wtCl1 Cas9.
Fig. 8 - Fifth generation of engineered Cl1pCas9 enzymes compared in terms of editing reporter activation. Representative previous generation Cl1pCas9 variants shown for reference. A) Raw luminescence signal. B) Fold change of signal (editing) compared to wtCl1 Cas9.
Fig. 9 - Sixth generation of engineered Cl1pCas9 enzymes compared in terms of editing reporter activation. Representative previous generation Cl1pCas9 variants shown for reference. A) Raw luminescence signal. B) Fold change of signal (editing) compared to wtCl1 Cas9.
Fig. 10 - Editing of the PCSK9 gene by representative Cl1pCas9 variants evaluated by next-generation sequencing. Well characterized ePsCas9 and SpCas9 with their corresponding guide RNAs are also shown.
Fig. 11-15 - Editing reporter activation by v4 Cl1pCas9 fused to different NLS combinations. A) Raw luminescence signal. B) Fold change of signal (editing) compared to wtcl I Cas9 with no NLS (nl0).
Fig. 16 - Editing reporter activation by wtCl1 pCas9 and engineered guide RNAs. A) Raw luminescence signal. B) Fold change of signal (editing) compared to wtCl1Cas9.
Fig. 17 - Editing reporter activation by v376 Cl1pCas9 and engineered guide RNAs. A) Raw luminescence signal. B) Fold change of signal (editing) compared to wtCl1Cas9.

### DETAILED DESCRIPTION OF THE INVENTION

The present disclosure provides a surprisingly highly active and improved Cas9 enzyme. Furthermore, said improved Cas9 enzyme is significantly smaller than Cas9 enzymes typically used in the field, such as SpCas9 or ePsCas9, which advantageously provides a Cas9 enzyme suitable for packaging and delivering to a cell, for instance by AAV delivery.

The inventors have surprisingly found that by mutating specific amino acid residue(s) within Cl1Cas9 protein sequence, a substantially inactive wild-type enzyme may be transformed into an extremely active nuclease. Besides having an enhanced nuclease activity, it has also been surprisingly found that said Cas9 protein also comprises an improved fidelity, which may further improve therapeutic uses in a subject, such as a human patient. Furthermore, the highly active Cl1Cas9 proteins are significantly smaller than traditional Ca9 proteins, thereby advantageously enabling AAV delivery.

Unless otherwise defined herein, scientific and technical terms used in the present disclosure shall have the meanings that are commonly understood by one of ordinary skill in the art. Further, unless otherwise required by context, singular terms shall include pluralities and plural terms shall include the singular. As used herein, "a" or "an" may mean one or more. As used herein, when used in conjunction with the word "comprising," the words "a" or "an" may mean one or more than one. As used herein, "another" or "a further" may mean at least a second or more.

The terms "peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

The terms "Cl1Cas9" and "Cl1pCas9" are used interchangeably and refer to a Cas9 protein expressed by *Campylobacter lanienae.*

The term "amino acid modification" refers to any amino acid modification, including amino acid substitution(s) and/or any amino acid post-translational modification.

Percent identity of polynucleotides or polypeptides can be determined when the polynucleotide or polypeptide sequences are aligned over a specified comparison window. In some embodiments, only specific portions of two or more sequences are aligned to determine sequence identity. In some embodiments, only specific domains of two or more sequences are aligned to determine sequence similarity. A comparison window can be a segment of at least 10 to over 1000 residues, at least 20 to about 1000 residues, or at least 50 to 500 residues in which the sequences can be aligned and compared. Methods of alignment for determination of sequence identity are well-known and can be performed using publicly available databases such as BLAST. For example, in some embodiments, "percent identity" of two amino acid sequences is determined using the algorithm of Karlin and Altschul, Proc Nat Acad Sci USA 87:2264-2268 (1990), modified as in Karlin and Altschul, Proc Nat Acad Sci USA 90:5873-5877 (1993). Such algorithms are incorporated into BLAST programs, e.g., BLAST+ or the NBLAST and XBLAST programs described in Altschul et al., J Mo! Biol, 215: 403-410 (1990). BLAST protein searches can be performed with programs such as, e.g., the XBLAST program, score=50, wordlength=3 to obtain amino acid sequences homologous to the protein molecules of the disclosure. Where gaps exist between two sequences, Gapped BLAST can be utilized as described in Altschul et al., Nucleic Acids Res 25(17): 3389-3402 (1997). When utilizing BLAST and Gapped BLAST programs, the default parameters of the respective programs (e.g., XBLAST and NBLAST) can be used.

As used herein, the terms "comprising" (and any variant or form of comprising, such as "comprise" and "comprises"), "having" (and any variant or form of having, such as "have" and "has"), "including" (and any variant or form of including, such as "includes" and "include") or "containing" (and any variant or form of containing, such as "contains" and "contain") are inclusive or open-ended and do not exclude additional, unrecited, elements or method steps.

As used herein, a "Cas protein" encompasses both Cas nucleases and Cas nickases. Cas proteins are part of the CRISPR/Cas system described herein. CRISPR/Cas systems, which include a Cas protein and a polynucleotide (also referred to as a "guide polynucleotide"), can be utilized for site-specific genome modifications.

As used herein, the term "enhanced endonuclease activity" refers to a Cas9 protein having a substantially higher endonuclease activity compared to a Cas9 protein in an unmodified state. Said endonuclease activity may be measured using the methods as described herein.

As used herein, the term "enhanced fidelity" refers to a Cas9 protein having a substantially higher fidelity compared to a Cas9 protein in an unmodified state. Said enhanced fidelity may refer to a Cas9 enzyme comprising one or more of the following: lower undesired insertion(s) and/or deletion(s), lower off-target cutting of DNA, less tolerance to DNA mismatches.

As used herein, the term "modification" of a target sequence encompasses single-nucleotide substitutions, multiple-nucleotide substitutions, insertions (i.e., knock-in) and deletions (i. e., knock-out) of a nucleic acid, frameshift mutations, and other nucleic acid modifications.

Following initial publications around the CRISPR-Cas9 system (Type II system), Cas9 variants have been identified in a range of bacterial species and a number have been functionally characterized. See, e.g., Chylinski et al., "Classification and evolution of type II CRISPR-Cas systems", Nucleic Acids Research 42(10): 6091-6105 (2014), Ran et al., "In vivo genome editing using Staphylococcus aureus Cas9", Nature 520(7546): 186-91 (2015), and Esvelt et al., "Orthogonal Cas9 proteins for RNA-guided gene regulation and editing", Nature Methods 10(11): 1116-1121 (2013), each of which is incorporated by reference herein in its entirety.

The present document encompasses novel Cas9 proteins having an enhanced activity and/or fidelity.

According to one embodiment, the wild-type Cl1Cas9 protein is as defined in Table 1 or SEQ ID NO: 1.

According to another embodiment, said Cas9 protein further comprises at least one nuclear localization signal (NLS), such as VC, 53BP1, bpSV40, BRCA1_1, BRCA1 2, EWS, FUS, HIV-I_TAT, hnRNP-D, hnRNP-M, HsNPM2, HTLV-1, mpSV40, MYC, NLP, NPM2, PTHrP and/or REV_HV1H2. Advantageously, the present document shows that an enhanced Cas9 enzyme may have its activity significantly improved by addition of at least one, such as two, NLS at the N and/or C-terminal portion of the enzyme.

Another aspect of the disclosure relates to a CRISPR-Cas system comprising a Cas9 protein comprising a polypeptide sequence having at least 95% identity, such as 97%, 98% or 99%, to SEQ ID NO: 1, wherein said polypeptide sequence comprises or consists of an amino acid modification at one or more positions of 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229, or a Cas9 protein as defined elsewhere herein; and a guide polynucleotide comprising a guide sequence, wherein the guide sequence is capable of hybridizing with a target sequence in a eukaryotic cell. In some embodiments, said Cas9 protein is as defined in Table 2 or said Cas9 protein comprises or consists of an amino acid sequence as defined in any one of SEQ ID NOs: 9-191, such as SEQ ID NOs: 9-144, SEQ ID NOs: 14-144, SEQ ID NOs: 30-144, SEQ ID NOs: 60-144, SEQ ID NOs: 112-144 and/or SEQ ID NOs: 140-144.

According to one embodiment, the guide polynucleotide comprises or consists of a polynucleotide sequence as defined in any one of SEQ ID NO: 192-206. Advantageously, the present document shows that a CRISPR-Cas system comprising an enhanced Cas9 enzyme may have its activity significantly improved by combining the enzyme with mutated gRNA sequences, such as the gRNA sequences as defined in any one of SEQ ID NO: 192-206.

**Table 1 - Wild-type Cl1Cas9 protein sequence**

| **SEQ ID NO 1 - Full sequence** |
|---|
| KSLSINIDMGAINNGIFIATCQDDKIIDKNAFDIKFDKGKLNFSKIDRTLRRHTRRSFDRDRFVLRLLGEILPLENLSNNQ KEKLFGLFKNRGFNYLNVNFNAEIDSDNLEILNALDRYYFKDCRVLSDFENSISKIFNELENNEILAFLDDQAQILNQA KEHKNKILNKNINAILEFLESIKNESFKNNKHRKSYLKDIKNEIENSFKDIILKLKIFKNSQELYYFIGNIANFQTRILRRYF NAKFDLVFDDAKLKKNILRNINYLDFISTDEKANKNKILENLNAFSTLEFLKNTDPIVTIPPYENAKNKKPQSCQALVIN DEKITPSLKDIAHKILSNSNFECLKTDENGVLADNIKDNEIAKYLQRILDISKGILDDKELYPRLLEKDENAKIFAQEIRLN SVELKEFKDFAKKYYDEVNLAKKGIIKNELLKPCCLHTPHKNKNKNALISALFLKSITQKEIENLEDFFANNKISGNQTFK GFFERLSELKKRYQNRFFHILEHEPTKELEGILKNYKKVMEKIVEHNSNFSFKAPFDDSTISTNFNYLTQLGDIIFDEKSR GFAKTCKFHTLENLIRSSAKSAIASRLASNSARLINGKIEMYLDRLAHEIIKKIDFTDIYNVQINVEMNKFNFESNLSKLG LAKERQKDEESFICPYSGAKISQTNCEYDHILPRSKGLYNSLANLIPTSSTSNQIKSNTKYLLSNLDENYLKYIFAKTQTTD LNSFKAFIDENISKIDENDFTNFKNLSFFEQIAFRHALFLDTDDNSFKKALRLLKLDTIKTHSNGTQKRFINILIEKISKLTK SGIDLNFIDSKLVSSTRKDLAQNDTSIAKQEIQDSHSHCVDASIVFYLANSKIIKGQIEPDYDYFKDIYPKDSDIILPQSKQ YLELNQKSQKSKKLFDDSVYSLVYEGKFDEKQKKILTQYELLKENGFVDTHKVFELIFKSFKDKDTIVLKDLKFLDNHLKS YIRKDIFSIFFNEKGELKEAKELKSATKNIDKFYTILSQNQDKIYTIKLDEKAKEKKTLQRNKVIELYKEIFYTKNAKPRARG KNRVTFSLPVYAKDAKYAIKRNSGFAGLKNKDIATKTYIDIDSREILKIPYYSTNVLPIRISDILEILSLQSKNAKQIYKIKIQ DNLPKQIVSLDFILSQANRHEVLVSFDKSKLEIQNINDDNGENTFIQKYLCDDFKEFLGKPRDNKIEIIKNNDDILAIKYC VESTSKANKEIIVKALSDETPSA |

Another aspect of the disclosure relates to a method for providing site-specific modification of a target sequence in a eukaryotic cell, the method comprising a) introducing into the eukaryotic cell a nucleotide sequence encoding a guide polynucleotide that forms a complex with a Cas9 protein and comprises a guide sequence, wherein the guide sequence is capable of hybridizing with a target sequence in host polynucleotide; and wherein the eukaryotic cell comprises the Cas9 protein as defined above; b) generating cohesive ends in the host polynucleotide with the Cas9 protein and the guide polynucleotide; and c) ligating i) the cohesive ends of (b) together, or ii) a 3' end of a polynucleotide sequence of interest to a first cohesive end of the host polynucleotide, and a 5' end of the polynucleotide sequence of interest to a second cohesive end of the host polynucleotide; thereby modifying the target sequence.

A "modification" of a target sequence encompasses single-nucleotide substitutions, multiple-nucleotide substitutions, insertions (i.e., knock-in) and deletions (i. e., knock-out) of a nucleic acid, frameshift mutations, and other nucleic acid modifications.

In embodiments of the method, the eukaryotic cell is an animal or human cell. In embodiments, the eukaryotic cell is an animal cell as described herein. In embodiments, the eukaryotic cell is a human cell. In embodiments, the eukaryotic cell is a human cell as described herein. In embodiments, the eukaryotic cell is a plant cell.

In embodiments of the method, the modification is deletion of at least part of the target sequence. In embodiments, the modification is mutation of the target sequence. In embodiments, the modification is inserting a sequence of interest into the target sequence. In embodiments, the modification is a modification as described herein.

### EXAMPLES

### Example 1 - HEK293T Cell Genome Editing Reporter Assay

The editing reporter system was generated by using the Xential method as per Li S et al., Nat Commun 12, 497 (2021) (which is incorporated by reference herein in its entirety) on wild type HEK293T cells. Briefly, a reporter cassette coding for a constitutively expressed gene was integrated into the HBGEF locus. The expressed gene terminates with an array of stop codons flanked by short repeats and a downstream non-expressed NanoLuc^{®} Luciferase gene. The sequence of the reporter cassette integrated into the HBEGF locus is as defined in SEQ ID NO: 209.

For reporter activation assays, the Xential reporter cell line was transfected with plasmids expressing Cas9 variants fused with designated nuclear localization sequences. Transfections were conducted by introducing a 5 µL mix containing Opti-MEM^{™} (Thermo), 50 ng plasmid and 0.3 µL FuGene (Promega) to each well containing 0.3 x 10^6 cells in a 96 well format, following the FuGene reagent use guidelines. After 24h, a designated amount of guide RNA targeting the stop codon array within the reporter cassette was introduced by using Lipofectamine^{™} RNAiMAX Transfection Reagent (Thermo) according to manufacturer's instructions.

Unless stated otherwise, guide RNA sequences (spacer underlined) used for targeting the integrated reporter cassette were 
CGAUUACCCUGUUAUCCCUACUGUUUCAAACCUUCGGCGUUUGAAAU
CACCACAAGGUGUCUUCAUAUCGGGGCAACUGCCUUUGGCACCCCCG
GUUA (SEQ ID NO: 210),
 
GUGCGCGCCGAGUAGGGAUAACGUUUCAGUUGAAAGAAGUCACUCU
UAAAGUGAGCUGAAAUCACUAAAAAUUAAGAUUGAACCCGGCUACU
GACUCUGUCAUCCGGGU (SEQ ID NO: 211),
 AUUACCCUGUUAUCCCUACUGUUUUAGAGCUAGAAAUAGCAAGUUA AAAUAAGGCUAGUCCGUUAUCAACUUGAAAAAGUGGCACCGAGUCG GUGCUUUU (SEQ ID NO: 212) for Cl1pCas9s, ePsCas9 and SpCas9, respectively. All guides were synthesized as end protected by having 2'-O-methylated bases and phosphorothioated bonds in first and last 3 positions.

Introduced guide RNA causes Cas9 mediated cleavage of the stop codon array within the reporter cassette, which in case of microhomology mediated end joining repair leads to the expression of NanoLuc^{®} that is secreted into the medium. 72 hours post guide RNA transfection, media was exchanged to a fresh one followed by 3-6h incubation. Equal amounts of media from treated reporter cells and Nano-Glo^{®} Luciferase substrate (Promega) diluted at 1:1000 in PBS were mixed and were immediately subjected to a luminescence readout on PheraStar FSX plate reader (BMG Labtech). This method allows for a luminescence represented readout of Cas9 mediated gene editing in cells.

Editing activity of Cl1pCas9 variants was compared to wtCl1Cas9 using this system. All variants including the wtCl1Cas9 were fused to N-terminal bpSV40 and C-terminal mpSV40 nuclear localization sequences unless specifically designated otherwise. This system was also used to assess the functionality of different Cl1pCas9 guide designs.

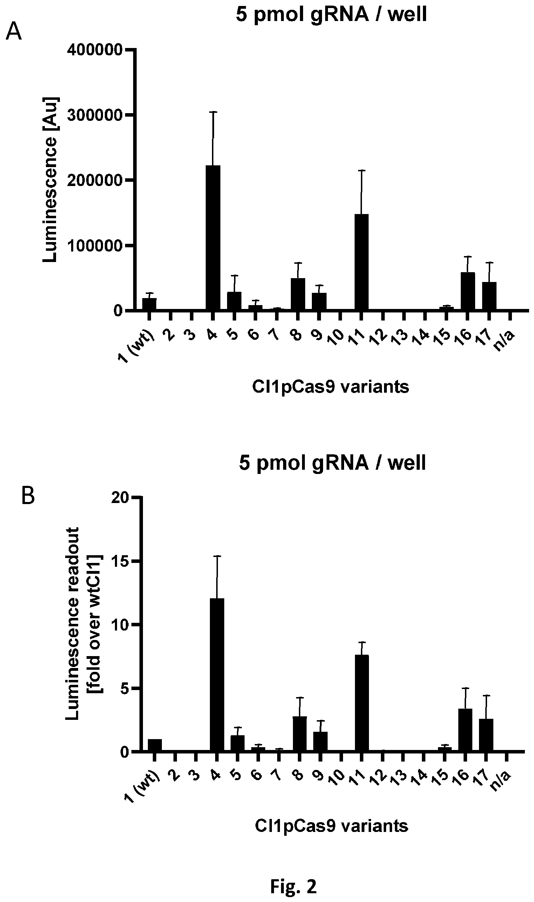

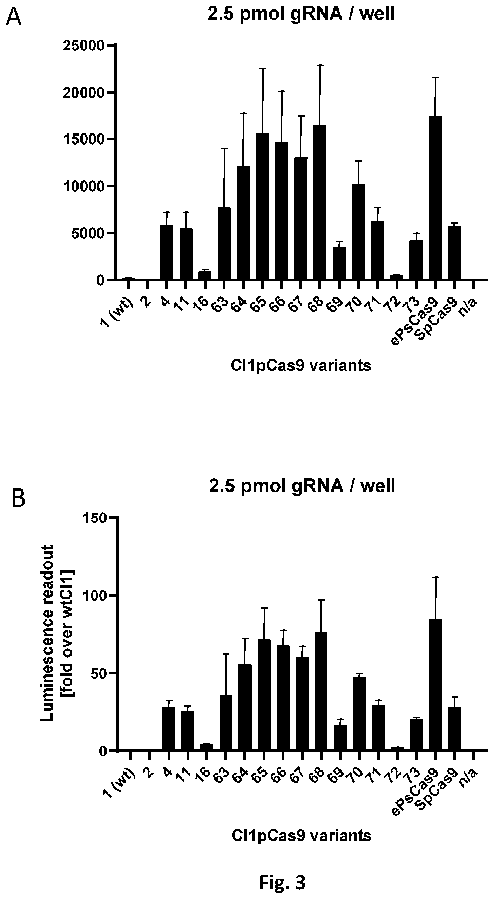

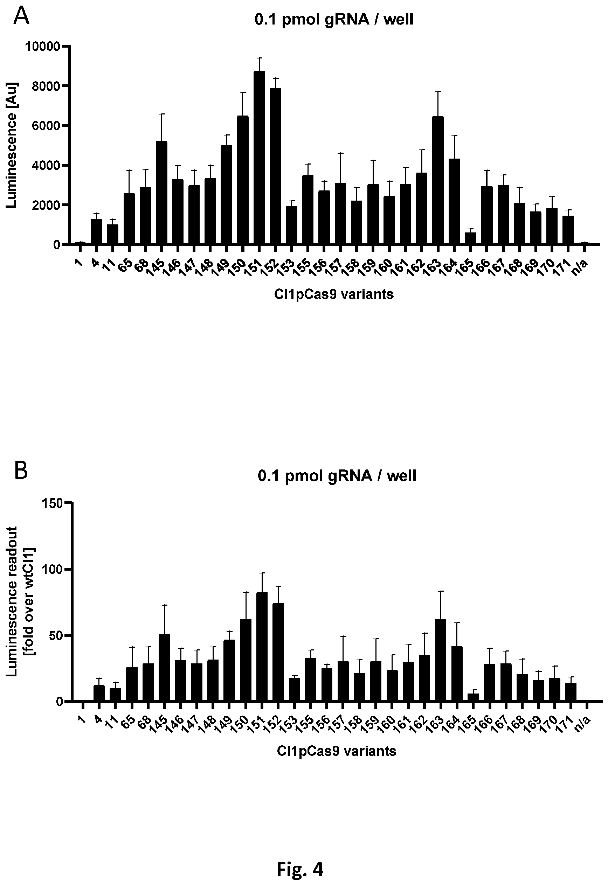

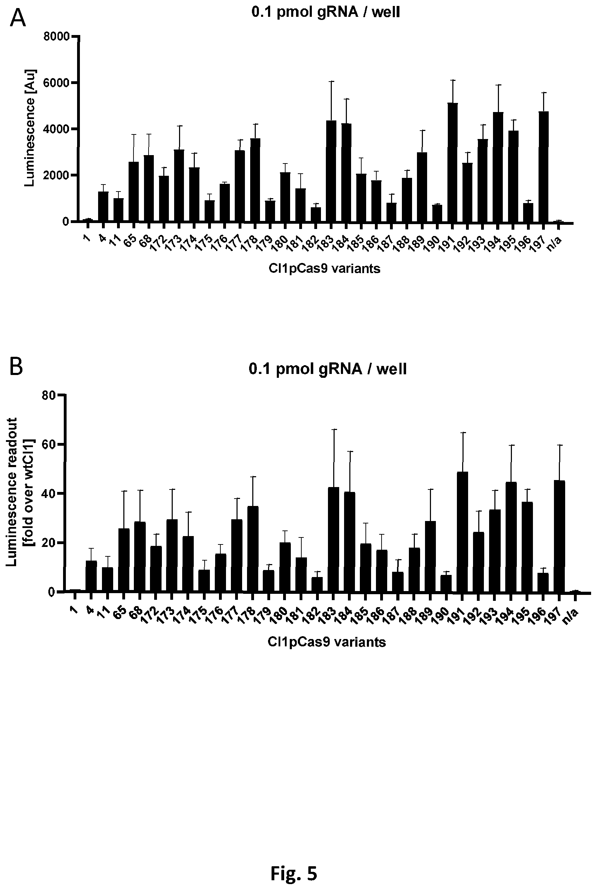

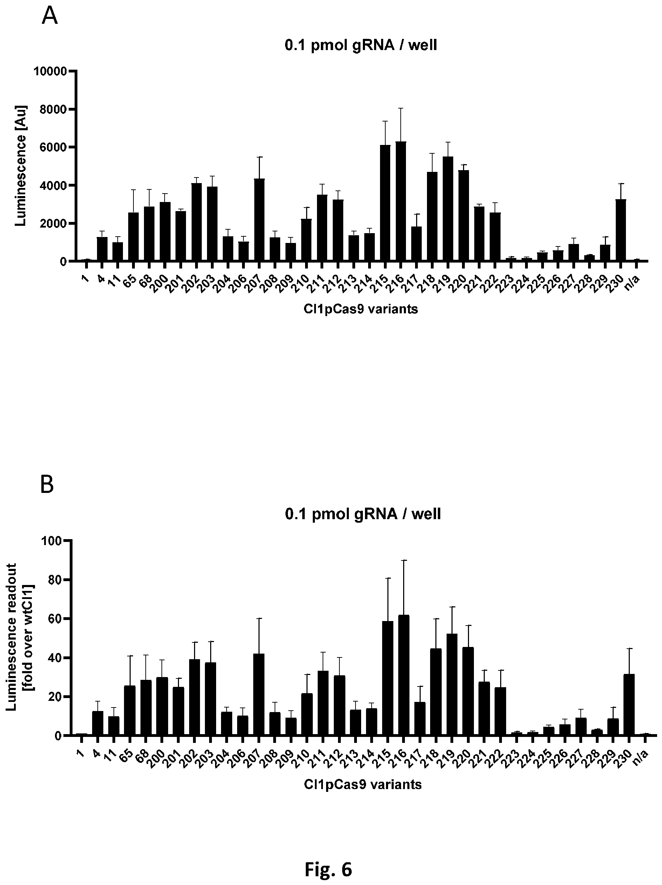

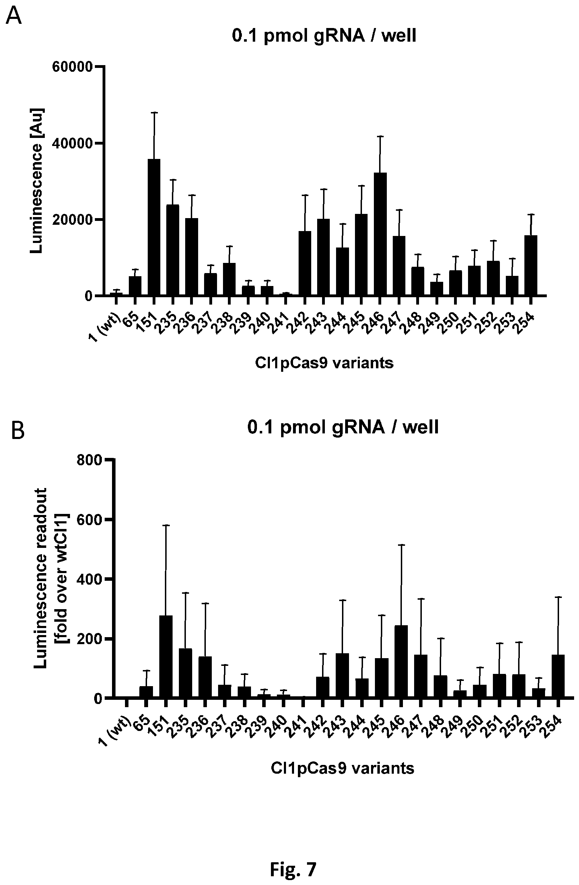

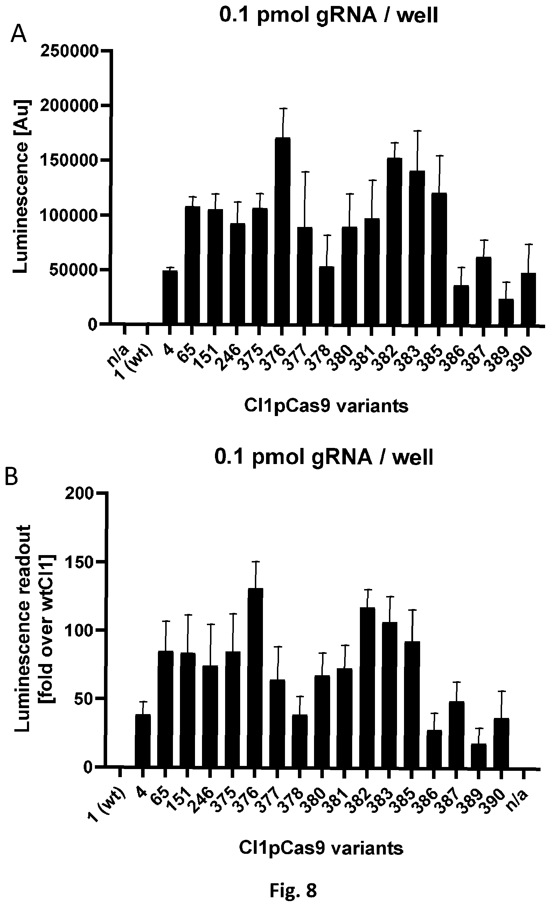

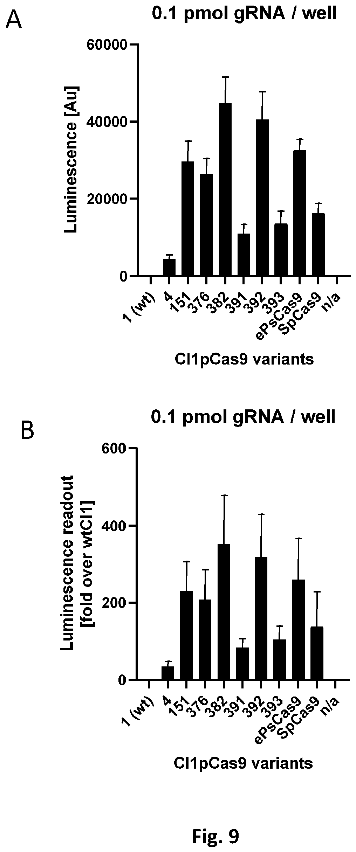

The editing activity is shown in Table 2. On each generation (GEN), a number of mutations are screened, based on the Cl1Cas9 proteins obtained in the previous generation. Generation "0" is the wild type Cl1Cas9 protein. It is clear from the results that the mutations that generated an improved enzyme were not predictable. In fact, some mutations may turn an improved enzyme of a previous generation into a non-active enzyme in the next generation. As shown in Figures 2-10, modified versions of the Cl1Cas9 enzyme are extremely more active (from dozens to more than a hundred times more active) than the wild-type Cl1Cas9 enzyme. Furthermore, as shown in Figures 3, 9 and 10, some Cl1Cas9 variants are at least as active as a highly optimized enzyme known in the art, namely ePsCas9 (also known as eSpotON). Obtaining an enzyme which is at least as active (or more active) as ePsCas9 and which is also significantly smaller is particularly advantageous, for example in the context of gene therapy, where it is easier and more effective to package and deliver a smaller Cas9 enzyme into the cell, for instance using AAV delivery.

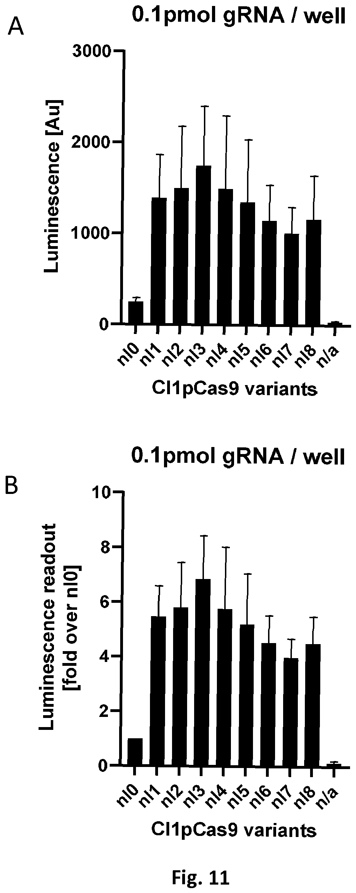

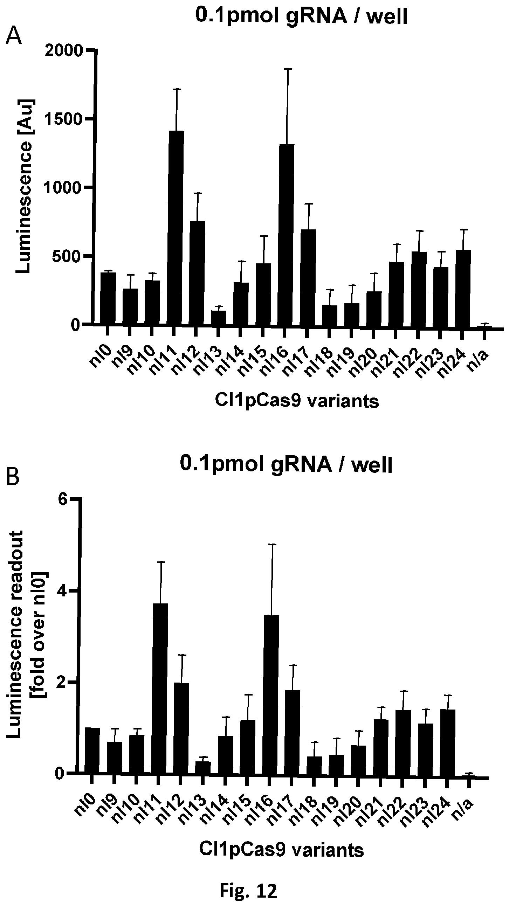

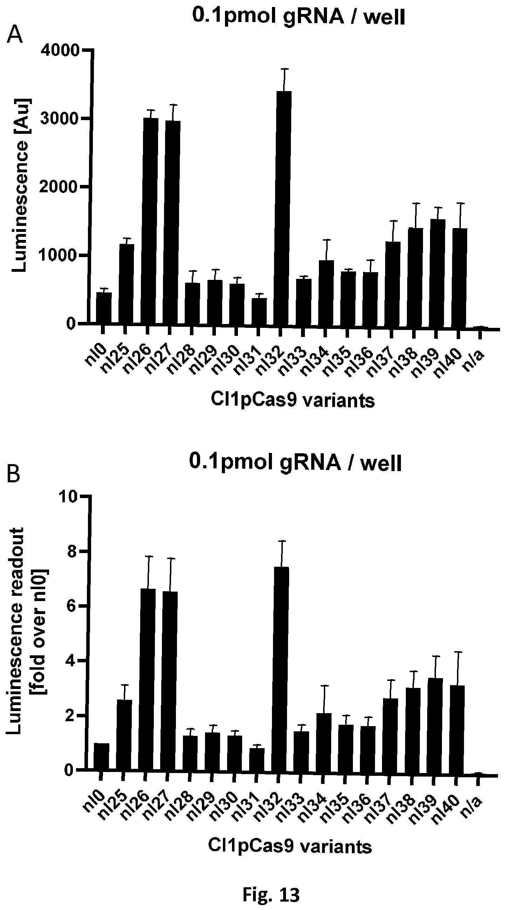

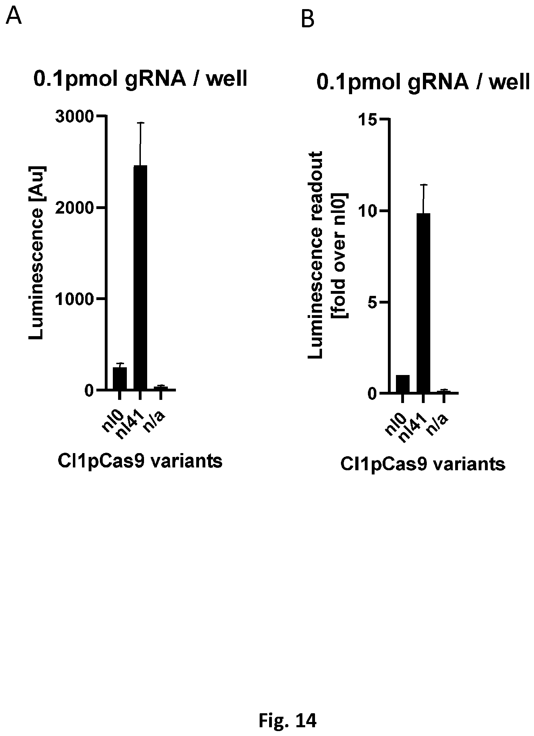

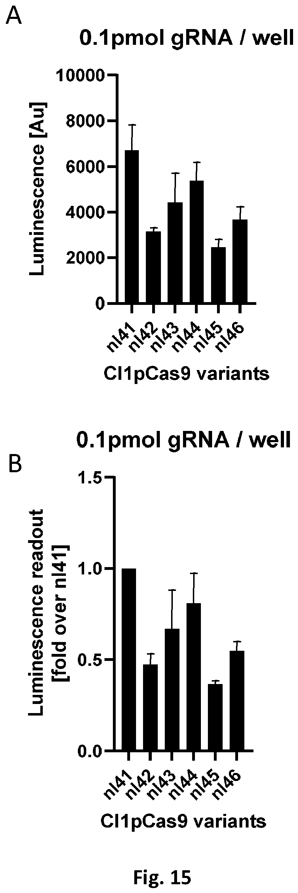

As shown in Table 3 and Figures 11-15, the editing activity of the Cas9 enzyme is significantly improved by the selection of one or more NLS to be attached to the N- and/or C-terminal end of the Cas9 enzyme. Some NLS and/or combinations thereof are particularly advantageous, for instance the ones selected from SEQ ID NO: 148-191, SEQ ID NO: 161-191, SEQ ID NO: 168-191, SEQ ID NO: 173-191, SEQ ID NO: 178-191, SEQ ID NO: 182-191, SEQ ID NO: 185-191 and/or SEQ ID NO: 189-191. Variant 4 of the Cl1Cas9 has been used merely as an exemplary proof of concept, and the skilled person would expect that a similar improvement is achieved when combining the above NLS with any other Cl1Cas9 variant disclosed herein.

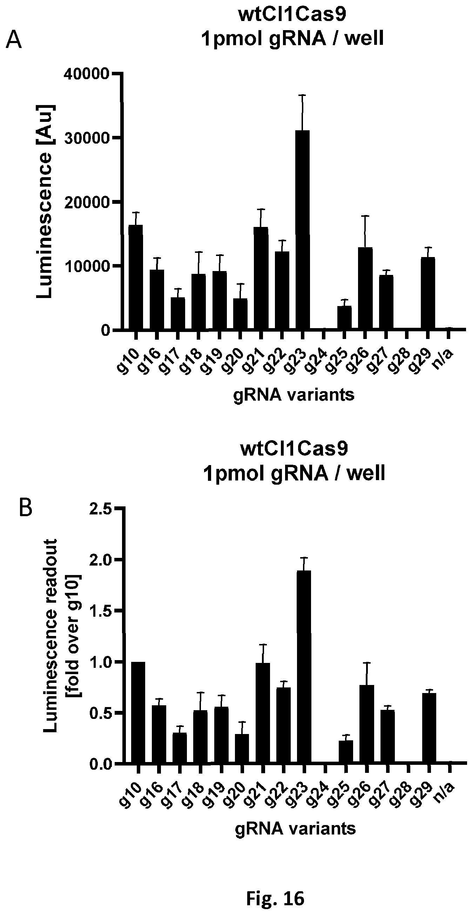

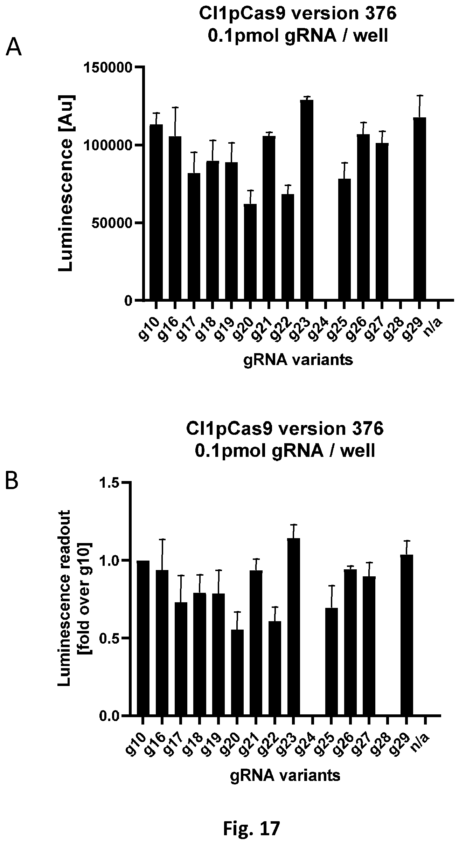

In some embodiments, guide RNAs have been modified to improve the activity of the CRISPR-Cas system. Methods for modifying gRNAs are known in the art, for example in Riesenberg et al (Nature communications, 2021), which is incorporated by reference in its entirety. Improved gRNAs are shown in Table 4, Figures 16-17 and SEQ ID NO: 192-206. SEQ ID NO: 207 refers to the spacer sequence comprised in each of the gRNAs. Said gRNA are capable of significantly increasing the activity of the Cl1Cas9, in particular the gRNA as defined in SEQ ID NO: 192, 198, 200 and/or 206. Variant 4 of the Cl1Cas9 has been used merely as an exemplary proof of concept, and the skilled person would expect that a similar improvement is achieved when combining the above gRNAs with any other Cl1Cas9 variant disclosed herein

**Table 2 - Cl1Cas9 variants**

| **SEQ ID NO** | **GEN** | **Cl1pCas9 variant #** | **HEK293T Editing Reporter Activation** | **1** | **2** | **3** | **4** | **5** | **6** | **7** |
|---|---|---|---|---|---|---|---|---|---|---|
| **1** | 0 | 1 (wt) | * | | | | | | | |
| **2** | 1 | 3 | - | H678A | | | | | | |
| **3** | 1 | 10 | - | N1089R | | | | | | |
| **4** | 1 | 12 | - | I1092R | | | | | | |
| **5** | 1 | 13 | - | A1093R | | | | | | |
| **6** | 1 | 14 | - | A1093K | | | | | | |
| **7** | 1 | 15 | - | A1160R | | | | | | |
| **8** | 1 | 2 | - | D8A | | | | | | |
| **9** | 1 | 6 | * | D911R | | | | | | |
| **10** | 1 | 7 | * | D911K | | | | | | |
| **11** | 2 | 72 | * | D37R | D1091R | D912R | | | | |
| **12** | 3 | 224 | * | D37R | D912R | N501R | | | | |
| **13** | 4 | 241 | * | D37R | N1161R | D1091S | S1229R | D912R | | |
| **14** | 1 | 5 | ** | G39R | | | | | | |
| **15** | 1 | 8 | ** | D912R | | | | | | |
| **16** | 1 | 9 | ** | 1978R | | | | | | |
| **17** | 1 | 16 | ** | N1161R | | | | | | |
| **18** | 1 | 17 | ** | S1229R | | | | | | |
| **19** | 2 | 69 | ** | D1091R | D912R | | | | | |
| **20** | 2 | 73 | ** | D37R | D1091R | S1229R | | | | |
| **21** | 3 | 165 | ** | D37R | S1229R | D1091R | | | | |
| **22** | 3 | 175 | ** | D37R | G39R | D1091R | | | | |
| **23** | 3 | 182 | ** | D1091R | N1161R | D912R | | | | |
| **24** | 3 | 187 | ** | D37R | D912R | D47R | | | | |
| **25** | 3 | 190 | ** | D37R | D912R | S57L | | | | |
| **26** | 3 | 225 | ** | D37R | D912R | N501F | | | | |
| **27** | 3 | 226 | ** | D37R | D912R | N501Y | | | | |
| **28** | 5 | 386 | ** | D37R | D912R | D1091S | I46R | | | |
| **29** | 5 | 389 | ** | D37R | D912R | D1091S | Q556R | | | |
| **30** | 1 | 4 | *** | D37R | | | | | | |
| **31** | 1 | 11 | *** | D1091R | | | | | | |
| **32** | 2 | 63 | *** | D37R | D1091R | | | | | |
| **33** | 2 | 71 | *** | D37R | D1091R | N1161R | | | | |
| **34** | 3 | 153 | *** | D37R | N1161R | D1091R | | | | |
| **35** | 3 | 168 | *** | D37R | G39R | D912G | | | | |
| **36** | 3 | 169 | *** | D37R | G39R | D912S | | | | |
| **37** | 3 | 170 | *** | D37R | G39R | D912N | | | | |
| **38** | 3 | 171 | *** | D37R | G39R | D912R | | | | |
| **39** | 3 | 176 | *** | D1091R | N1161R | S1229R | | | | |
| **40** | 3 | 179 | *** | D1091R | N1161R | D912G | | | | |
| **41** | 3 | 181 | *** | D1091R | N1161R | D912N | | | | |
| **42** | 3 | 196 | *** | D37R | D912R | D61L | | | | |
| **43** | 3 | 204 | *** | D37R | D912R | D911G | | | | |
| **44** | 3 | 206 | *** | D37R | D912R | D911S | | | | |
| **45** | 3 | 208 | *** | D37R | D912R | D1073G | | | | |
| **46** | 3 | 209 | *** | D37R | D912R | D1073A | | | | |
| **47** | 3 | 210 | *** | D37R | D912R | E508G | | | | |
| **48** | 3 | 213 | *** | D37R | D912R | E1164R | | | | |
| **49** | 3 | 214 | *** | D37R | D912R | S1158N | E1164R | | | |
| **50** | 3 | 217 | *** | D37R | D912R | D1206R | | | | |
| **51** | 3 | 227 | *** | D37R | D912R | N501W | | | | |
| **52** | 3 | 229 | *** | D37R | D912R | F94G | | | | |
| **53** | 4 | 239 | *** | D37R | N1161R | D1091S | S1229R | D912S | | |
| **54** | 4 | 240 | *** | D37R | N1161R | D1091S | S1229R | D912N | | |
| **55** | 4 | 249 | *** | D37R | N1161R | D1091S | D1206S | D912R | | |
| **56** | 4 | 253 | *** | D37R | N1161R | D1091S | E1226G | D1206S | | |
| **57** | 5 | 378 | *** | D37R | N1161R | D1091S | D1206S | S1229R | | |
| **58** | 5 | 387 | *** | D37R | D912R | D1091S | D1206S | | | |
| **59** | 5 | 390 | *** | D37R | D912R | D1091S | S566R | | | |
| **60** | 2 | 64 | **** | D37R | N1161R | | | | | |
| **61** | 2 | 65 | **** | D37R | D912R | | | | | |
| **62** | 2 | 66 | **** | D37R | S1229R | | | | | |
| **63** | 2 | 67 | **** | D37R | G39R | | | | | |
| **64** | 2 | 68 | **** | D1091R | N1161R | | | | | |
| **65** | 2 | 70 | **** | D1091R | S1229R | | | | | |
| **66** | 3 | 146 | **** | D37R | N1161R | D912G | | | | |
| **67** | 3 | 155 | **** | D37R | D912R | S1229R | | | | |
| **68** | 3 | 156 | **** | D37R | D912R | D1091G | | | | |
| **69** | 3 | 157 | **** | D37R | D912R | D1091S | | | | |
| **70** | 3 | 158 | **** | D37R | D912R | D1091N | | | | |
| **71** | 3 | 159 | **** | D37R | S1229R | D912G | | | | |
| **72** | 3 | 160 | **** | D37R | S1229R | D912S | | | | |
| **73** | 3 | 161 | **** | D37R | S1229R | D912N | | | | |
| **74** | 3 | 162 | **** | D37R | S1229R | D1091G | | | | |
| **75** | 3 | 164 | **** | D37R | S1229R | D1091N | | | | |
| **76** | 3 | 166 | **** | D37R | G39R | N1161R | | | | |
| **77** | 3 | 167 | **** | D37R | G39R | S1229R | | | | |
| **78** | 3 | 172 | **** | D37R | G39R | D1091G | | | | |
| **79** | 3 | 173 | **** | D37R | G39R | D1091S | | | | |
| **80** | 3 | 174 | **** | D37R | G39R | D1091N | | | | |
| **81** | 3 | 177 | **** | D1091R | N1161R | D37G | | | | |
| **82** | 3 | 178 | **** | D1091R | N1161R | D37N | | | | |
| **83** | 3 | 180 | **** | D1091R | N1161R | D912S | | | | |
| **84** | 3 | 185 | **** | D37R | D912R | D47G | | | | |
| **85** | 3 | 186 | **** | D37R | D912R | D47L | | | | |
| **86** | 3 | 188 | **** | D37R | D912R | T54R | | | | |
| **87** | 3 | 189 | **** | D37R | D912R | S57A | | | | |
| **88** | 3 | 192 | **** | D37R | D912R | D59G | | | | |
| **89** | 3 | 193 | **** | D37R | D912R | D59S | | | | |
| **90** | 3 | 200 | **** | D37R | D912R | Q315K | | | | |
| **91** | 3 | 201 | **** | D37R | D912R | Q315R | | | | |
| **92** | 3 | 202 | **** | D37R | D912R | S566K | | | | |
| **93** | 3 | 203 | **** | D37R | D912R | S566R | | | | |
| **94** | 3 | 207 | **** | D37R | D912R | K980R | | | | |
| **95** | 3 | 211 | **** | D37R | D912R | E508R | | | | |
| **96** | 3 | 212 | **** | D37R | D912R | D1122R | | | | |
| **97** | 3 | 218 | **** | D37R | D912R | E1226G | | | | |
| **98** | 3 | 220 | **** | D37R | D912R | E1226R | | | | |
| **99** | 3 | 221 | **** | D37R | D912R | I978R | | | | |
| **100** | 3 | 222 | **** | D37R | D912R | A1160R | | | | |
| **101** | 4 | 237 | **** | D37R | N1161R | D1091S | D912R | | | |
| **102** | 4 | 238 | **** | D37R | N1161R | D1091S | S1229R | | | |
| **103** | 4 | 248 | **** | D37R | N1161R | D1091S | D1206S | D912N | | |
| **104** | 4 | 250 | **** | D37R | N1161R | D1091S | D1206S | S1229R | | |
| **105** | 4 | 251 | **** | D37R | N1161R | D1091S | E1226G | | | |
| **106** | 4 | 252 | **** | D37R | N1161R | D1091S | E1226S | | | |
| **107** | 5 | 377 | **** | D37R | N1161R | D1091S | I46R | S1229R | | |
| **108** | 5 | 380 | **** | D37R | N1161R | D1091S | I46R | G39R | | |
| **109** | 5 | 381 | **** | D37R | N1161R | D1091S | D1206S | G39R | | |
| **110** | 6 | 391 | **** | D37R | N1161R | D1091S | I46R | D1206S | S1229R | |
| **111** | 6 | 393 | **** | D37R | N1161R | D1091S | I46R | D1206N | S566R | D912N |
| **112** | 3 | 145 | ***** | D37R | N1161R | S1229R | | | | |
| **113** | 3 | 147 | ***** | D37R | N1161R | D912S | | | | |
| **114** | 3 | 148 | ***** | D37R | N1161R | D912N | | | | |
| **115** | 3 | 149 | ***** | D37R | N1161R | D912R | | | | |
| **116** | 3 | 150 | ***** | D37R | N1161R | D1091G | | | | |
| **117** | 3 | 151 | ***** | D37R | N1161R | D1091S | | | | |
| **118** | 3 | 152 | ***** | D37R | N1161R | D1091N | | | | |
| **119** | 3 | 163 | ***** | D37R | S1229R | D1091S | | | | |
| **120** | 3 | 183 | ***** | D37R | D912R | I46A | | | | |
| **121** | 3 | 184 | ***** | D37R | D912R | I46R | | | | |
| **122** | 3 | 191 | ***** | D37R | D912R | S57R | | | | |
| **123** | 3 | 194 | ***** | D37R | D912R | D59R | | | | |
| **124** | 3 | 195 | ********* | D37R | D912R | D61G | | | | |
| **125** | 3 | 197 | ********* | D37R | D912R | D61R | | | | |
| **126** | 3 | 215 | ********* | D37R | D912R | D1206G | | | | |
| **127** | 3 | 216 | ********* | D37R | D912R | D1206S | | | | |
| **128** | 3 | 219 | ********* | D37R | D912R | E1226S | | | | |
| **129** | 3 | 230 | ********* | D37R | D912R | F94Y | | | | |
| **130** | 4 | 235 | ********* | D37R | N1161R | D1091S | D912S | | | |
| **131** | 4 | 236 | ********* | D37R | N1161R | D1091S | D912N | | | |
| **132** | 4 | 242 | ********* | D37R | N1161R | D1091S | I46R | | | |
| **133** | 4 | 243 | ********* | D37R | N1161R | D1091S | S57R | | | |
| **134** | 4 | 244 | ********* | D37R | N1161R | D1091S | D59S | | | |
| **135** | 4 | 245 | ********* | D37R | N1161R | D1091S | S566R | | | |
| **136** | 4 | 246 | ********* | D37R | N1161R | D1091S | D1206S | | | |
| **137** | 4 | 247 | ********* | D37R | N1161R | D1091S | D1206S | D912S | | |
| **138** | 4 | 254 | ********* | D37R | N1161R | D1091S | F94Y | | | |
| **139** | 5 | 375 | ********* | D37R | N1161R | D1091S | I46R | D1206S | | |
| **140** | 5 | 376 | ********** | D37R | N1161R | D1091S | I46R | D1206N | | |
| **141** | 5 | 382 | ********** | D37R | N1161R | D1091S | S566R | I46R | | |
| **142** | 5 | 383 | ********** | D37R | N1161R | D1091S | S566R | D1206S | | |
| **143** | 5 | 385 | ********** | D37R | N1161R | D1091S | S566R | D912N | | |
| **144** | 6 | 392 | ********** | D37R | N1161R | D1091S | I46R | D1206N | S566R | |

**Table 3 - Cl1Cas9 and NLS**

| **SEQ ID NO** | **#** | **N-terminal** | | **Cl1pCas9 variant #** | **C-terminal** | | |
|---|---|---|---|---|---|---|---|
| | | **NLS** | **linker** | | **linker** | **NLS** | **Reporter activation** |
| **145** | nl13 | VC | 18 | **4** | | | - |
| **146** | nl18 | EWS | 18 | **4** | | | - |
| **147** | nl19 | hnRNP-M | 18 | **4** | | | - |
| **148** | nl0 | | | **4** | | | * |
| **149** | nl9 | mpSV40 | 18 | **4** | | | * |
| **150** | nl10 | MYC | 18 | **4** | | | * |
| **151** | nl14 | BRCA1_1 | 18 | **4** | | | * |
| **152** | nl15 | BRCA1_2 | 18 | **4** | | | * |
| **153** | nl20 | hnRNP-D | 18 | **4** | | | * |
| **154** | nl21 | HIV-I_TAT | 18 | **4** | | | * |
| **155** | nl22 | REV_HV1H2 | 18 | **4** | | | * |
| **156** | nl23 | HTLV-1 | 18 | **4** | | | * |
| **157** | nl28 | | | **4** | 18 | HsNPM2 | * |
| **158** | nl29 | | | **4** | 18 | VC | * |
| **159** | nl30 | | | **4** | 18 | BRCA1 1 | * |
| **160** | nl31 | | | **4** | 18 | BRCA1_2 | * |
| **161** | nl12 | HsNPM2 | 18 | **4** | | | ** |
| **162** | nl17 | FUS | 18 | **4** | | | ** |
| **163** | nl24 | PTHrP | 18 | **4** | | | ** |
| **164** | nl33 | | | **4** | 18 | FUS | ** |
| **165** | nl34 | | | **4** | 18 | EWS | ** |
| **166** | nl35 | | | **4** | 18 | hnRNP-M | ** |
| **167** | nl36 | | | **4** | 18 | hnRNP-D | ** |
| **168** | nl25 | | | **4** | 18 | m pSV40 | *** |
| **169** | nl37 | | | **4** | 18 | HIV-I_TAT | *** |
| **170** | nl38 | | | **4** | 18 | REV_HV1H2 | *** |
| **171** | nl40 | | | **4** | 18 | PTHrP | *** |
| **172** | nl45 | bpSV40 | 2 | **4** | 2 | NLP | *** |
| **173** | nl6 | | | **4** | 9 | bpSV40 | **** |
| **174** | nl7 | | | **4** | 18 | bpSV40 | **** |
| **175** | nl11 | NLP | 18 | **4** | | | **** |
| **176** | nl16 | 53BP1 | 18 | **4** | | | **** |
| **177** | nl39 | | | **4** | 18 | HTLV-1 | **** |
| **178** | nl1 | bpSV40 | 2 | **4** | | | ***** |
| **179** | nl5 | | | **4** | 2 | bpSV40 | ***** |
| **180** | nl8 | | | **4** | 32 | bpSV40 | ***** |
| **181** | nl42 | NPM2 | 2 | **4** | 2 | bpSV40 | ***** |
| **182** | nl2 | bpSV40 | 9 | **4** | | | ****** |
| **183** | nl4 | bpSV40 | 32 | **4** | | | ****** |
| **184** | nl46 | bpSV40 | 2 | **4** | 2 | 53bp1 | ****** |
| **185** | nl3 | bpSV40 | 18 | **4** | | | ******* |
| **186** | nl26 | | | **4** | 18 | MYC | ******* |
| **187** | nl27 | | | **4** | 18 | NLP | ******* |
| **188** | nl43 | NLP | 2 | **4** | 2 | bpSV40 | ******* |
| **189** | nl32 | | | **4** | 18 | 53BP1 | ******** |
| **190** | nl44 | bpSV40 | 2 | **4** | 2 | MYC | ******** |
| **191** | nl41 | bpSV40 | 2 | **4** | 2 | mpSV40 | ********** |

**Table 4 - Guide RNAs**

| **SEQ ID NO** | # | HEK293T Editing Reporter Activation | Length [nt] | Scaffold lenght [nt] | Full sequence (5'-3') |
|---|---|---|---|---|---|
| **192** | g10 | ******* | 121 | 99 | CGAUUACCCUGUUAUCCCUACUGUUUCAAACCAAGUUGGAUUCAACAAUCUAGCUUAUAGCGUU UGAAAUCACCACAAGGUGUCUUCAUAUCGGGGCAACUGCCUUUGGCACCCCCGGUUA |
| **193** | g16 | ****** | 108 | 86 | CGAUUACCCUGUUAUCCCUACUGUUUCAAACCGGCACUUCGGUGCCGCGUUUGAAAUCACCACA AGGUGUCUUCAUAUCGGGGCAACUGCCUUUGGCACCCCCGGUUA |
| **194** | g17 | ***** | 106 | 84 | CGAUUACCCUGUUAUCCCUACUGUUUCAAACCGGACUUCGGUCCGCGUUUGAAAUCACCACAAG GUGUCUUCAUAUCGGGGCAACUGCCUUUGGCACCCCCGGUUA |
| **195** | g18 | ****** | 100 | 78 | CGAUUACCCUGUUAUCCCUACUGUUUCAAACCCUUCGGGCGUUUGAAAUCACCACAAGGUGUCU UCAUAUCGGGGCAACUGCCUUUGGCACCCCCGGUUA |
| **196** | g19 | ****** | 98 | 76 | CGAUUACCCUGUUAUCCCUACUGUUUCAAACCGAAAGCGUUUGAAAUCACCACAAGGUGUCUUC AUAUCGGGGCAACUGCCUUUGGCACCCCCGGUUA |
| **197** | g20 | ***** | 113 | 91 | CGAUUACCCUGUUAUCCCUACUGUUUCAAACCAGGCACUUCGGUGCCUAUAGCGUUUGAAAUCA CCACAAGGUGUCUUCAUAUCGGGGCAACUGCCUUUGGCACCCCCGGUUA |
| **198** | g21 | ******* | 111 | 89 | CGAUUACCCUGUUAUCCCUACUGUUUCAAACCAGGACUUCGGUCCUAUAGCGUUUGAAAUCACC ACAAGGUGUCUUCAUAUCGGGGCAACUGCCUUUGGCACCCCCGGUUA |
| **199** | g22 | ****** | 105 | 83 | CGAUUACCCUGUUAUCCCUACUGUUUCAAACCACUUCGGUAUAGCGUUUGAAAUCACCACAAGG UGUCUUCAUAUCGGGGCAACUGCCUUUGGCACCCCCGGUUA |
| **200** | g23 | ******** | 98 | 76 | CGAUUACCCUGUUAUCCCUACUGUUUCAAACCUUCGGCGUUUGAAAUCACCACAAGGUGUCUUC AUAUCGGGGCAACUGCCUUUGGCACCCCCGGUUA |
| **201** | g24 | | 95 | 73 | CGAUUACCCUGUUAUCCCUACUGUUUCAAACAAACGUUUGAAAUCACCACAAGGUGUCUUCAUA UCGGGGCAACUGCCUUUGGCACCCCCGGUUA |
| **202** | g25 | ***** | 105 | 83 | CGAUUACCCUGUUAUCCCUACUGUUUCAAACGGCACUUCGGUGCCGUUUGAAAUCACCACAAGG UGUCUUCAUAUCGGGGCAACUGCCUUUGGCACCCCCGGUUA |
| **203** | g26 | ****** | 97 | 75 | CGAUUACCCUGUUAUCCCUACUGUUUCAAACCUUCGGGUUUGAAAUCACCACAAGGUGUCUUCA UAUCGGGGCAACUGCCUUUGGCACCCCCGGUUA |
| **204** | g27 | ****** | 126 | 104 | CGAUUACCCUGUUAUCCCUACUGUUUCAAACCAAGUUGGAUUCAACAAUCUAGCUUAUAGCGUU UGAAAUCACCACAAGGUGUCUUCAUAUCGGGGCAACUGGCACUUCGGUGCCACCCCCGGUUA |
| **205** | g28 | | 124 | 102 | CGAUUACCCUGUUAUCCCUACUGUUUCAAACCAAGUUGGAUUCAACAAUCUAGCUUAUAGCGUU UGAAAUCACCACAAGGUGUCUUCAUAUCGGGGCAACUGGACUUCGGUCCACCCCCGGUUA |
| **206** | g29 | ******* | 122 | 100 | CGAUUACCCUGUUAUCCCUACUGUUUCAAACCAAGUUGGAUUCAACAAUCUAGCUUAUAGCGUU UGAAAUCACCACAAGGUGUCUUCAUAUCGGGGCAACUGCCUUCGGGCACCCCCGGUUA |
| **207** | spacer (5'-3') | - | 22 | | CGAUUACCCUGUUAUCCCUACU |

**Table 5 - eSPOTON protein sequence**

| SEQ ID NO: 208 - eSPOTON |
|---|
| MKRTADGSEFESPKKKRKVTGTVKSTLAVDMGGRYTGIFSYTTDSGFPKAKEARAYVLNMPDNDALTYSMAARTQTRHRIRSQQRFVLA RRLTYILIEGKLKRKLSPREKEAISSLLRRRGYSRLESELDLSVLQGVESGFFKCFLPNFDEDENLLTQWTSLTDGYLQNNSDSRRQIQIFLES SKDSKEFLTVVKSQHQDTKEYKNALKVMRDDAESMIEQSMFGHKHRRLYLEAIAQDIPRDSRLKPHEAFSGVEKFHHFIGNLSNLQLRALR WYFNDPSMKNNVFDKERLKSVLVRAYQFFHYPKDLTQQRAEVLNAYEGATDILETLQTLNPELTIPPYEDQNNRRPPLDQTLWLSPRLLD QRYGDTWEIWVQNLLRSPLSKGIDENLDTILITTDRKARLLERQSGRLIHYTSQKLYHSYVLQRLLDRTVENDAYLLKTLVSSNRGNSNEIH QAQERLTRDLGSQHIKKFLDFVRQYYDEVDKAKRGLWFIVEKPLMERADIHPPMKNDSVILRLVGNILCVSDLVDLSFWTRKVKGQSTVR SLCTAIEKTRKEYGNSFNYLYQRALYLQSKGKKLSAEDKDFIKLQSNVLLVSDVIAEALDIKEEQKKKFANPFSLAQLYNIIETEKSGFISTTL AAVDENAWRNNLQGKARCVQLCADTVRPFDGALRNILDRQAYEIAKLKAEELLSTELKNQTIDLVVLLESNQFAFSASLAEVKKSANTAA IRQKVAKAQKRQQDRWLSKDERIKSASRGLCPYTGKNLGDKGEVDHIIPRSLSMNYMGSILNSEANLIYCSQEGNQLKLNGRKKLSDLAD NYLKVVFGTADRGTICKYIEKSVSELTDAKIVQFELLDRSQQDAVRHALFLEDFSEARRRHRLLGKINTARVNGTQAWFAKSFITKLRELTK EWCANNQITLAFDLYRLDAQTVSQDYRKKFALINKDWAKPDDKKQPIASHAIDAFCVFAAAKDKRNIANVLGVFDEVAEEQNLKTIAQL MPSEVNLISPKRKSILDKNEVGSRALMKEGIFAEHFLPILVRGDDCRIGFDWSESGSVKVKDADKLFGVLDGLLKQSQKRSVNGFETYTVD RIKAFELLHDVFIRPCSQKMLEQAEVLEKLHYITQNISVTSVYDAVNRQFKCREEILKDKDFDIKVDLGNRFGSAKGKITLPAKREWEKLVN RSELKNLIKDKLSDKGSEKTPDGETLIYDIFRSIPVQKLSHKATRRVWSLPKIPSISSGVRIKRKDSNGNDIYQLYMLNDTKCKGFVVNEKGV IDWSSDLVADLYKQPTLTILNGRYLKADQYVRMDQWYEVDCGRDDVIVKMCPGTSGRRYIEITQSKKQFEDWTGYISGSFWNYPVTIKLS SQQIANFVKNSQMPLLGKPRSGQITVITLGNTLKYWYCVESKNSMMNEAYQKAYLVHFNQGTPKKKRKV |

### Example 2 - Editing Efficiency Analysis by Amplicon Sequencing

Wild-type HEK293T cells were treated identical as the reporter strain (described above) with the exception of using guide RNA targeting the designated gene.

Guide RNA sequences (spacer underlined) used for targeting the PCSK9 gene were 
UCCAGGUUCCACGGGAUGCUCUGUUUCAAACCUUCGGCGUUUGAAAUCA
CCACAAGGUGUCUUCAUAUCGGGGCAACUGCCUUUGGCACCCCCGGUUA
(SEQ ID NO: 213),
 
UCCAGGUUCCACGGGAUGCUCUGUUUCAGUUGAAAGAAGUCACUCUUAA
AGUGAGCUGAAAUCACUAAAAAUUAAGAUUGAACCCGGCUACUGACUC
UGUCAUCCGGGU (SEQ ID NO: 214)
 and CAGGUUCCACGGGAUGCUCUGUUUUAGAGCUAGAAAUAGCAAGUUAAA AUAAGGCUAGUCCGUUAUCAACUUGAAAAAGUGGCACCGAGUCGGUGC UUUU (SEQ ID NO: 215) for Cl1pCas9s, ePsCas9 and SpCas9, respectively. All guides were synthesized as end protected by having 2'-O-methylated bases and phosphorothioated bonds in first and last 3 positions.

Post 72h incubation step, genomic DNA was isolated using the QuickExtract DNA Extraction Solution (Lucigen) as per manufacturer's instructions in final volume of 50 µL.

Primary amplicons were generated using 0.25 µM target-specific primers (IDT), 1.5uL genomic DNA and the Phusion Flash High-Fidelity PCR Master Mix (Thermo) as per manufacturers guidelines. PCR reaction conditions used were 98°C for 1 min followed by 32 cycles of 98°C for 10 s, 60-65°C for 10 s, and 72°C for 10 s. PCR products were cleaned up with Ampure XP beads (Beckman) and analyzed using the Fragment Analyzer (Agilent). Indexing was performed by secondary PCR using 1 ng of the primary amplicon, 0.5 µM indexing primers (IDT) and KAPA HiFi HotStart Ready Mix (Roche) used as per manufacturer's instructions. Secondary PCR reaction conditions used were 72°C for 3 min; 98°C for 30 s; followed by 10 cycles of 98°C for 10 s, 63°C for 30 s, and 72°C for 3 min; followed by the final cycle of 72°C for 5 min. Cleanup and quality control were done identically as for the primary amplification.

Sequence library quantification was performed by using the Qubit 4 Fluorometer (Thermo). For sequencing the Illumina NextSeq platform was employed, as per manufacturer's guidelines. Data demultiplexing was performed by using bcl2fastq software. Editing efficiencies were obtained by applying the RIMA method as described in Taheri-Ghahfarokhi A., et al., Nucleic Acids Res 46(16):8417-8434 (2018*),* which is incorporated herein by reference in its entirety.

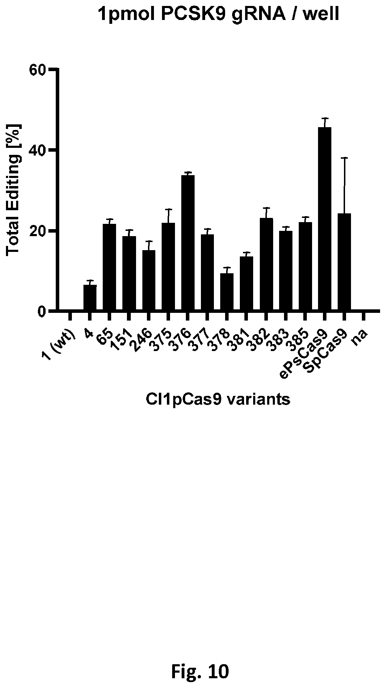

The editing results of the next-generation sequencing are shown in Figure 10. All references cited herein, including patents, patent applications, papers, textbooks and the like, and the references cited therein, to the extent that they are not already, are hereby incorporated herein by reference in their entirety.

## Claims

1. A Cas9 protein comprising a polypeptide sequence having at least 95% identity, such as 97%, 98% or 99%, to SEQ ID NO: 1, wherein said polypeptide sequence comprises an amino acid modification at one or more positions relative to SEQ ID NO: 1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229.

2. The Cas9 protein according to claim 1, wherein said Cas9 protein comprises an enhanced endonuclease activity compared to a wild type Cas9 protein, such as a wild type Cl1Cas9 protein.

3. The Cas9 protein according to any one of the preceding claims, wherein the polypeptide sequence comprises one or more amino acid modifications selected from D1073A, A1160R, D1073G, D1091G, D1091N, D1091R, D1091S, D1122R, D1206G, D1206N, D1206R, D1206S, D37G, D37N, D37R, D47G, D47L, D47R, D59G, D59R, D59S, D61G, D61L, D61R, D911G, D911K, D911R, D911S, D912G, D912N, D912R, D912S, E1164R, E1226G, E1226R, E1226S, E508G, E508R, F94G, F94Y, G39R, I46A, I46R, I978R, K980R, N1161R, N501F, N501R, N501W, N501Y, Q315K, Q315R, Q556R, S1229R, S566K, S566R, S57A, S57L, S57R, and T54R.

4. The Cas9 protein according to any one of the preceding claims, wherein the polypeptide sequence comprises a first amino acid modification selected from D37R, D1091R, D912R, G39R, I978R, N1161R, and S1229R.

5. The Cas9 protein according to claim 4, wherein the polypeptide sequence further comprises a second amino acid modification selected from D1091R, D912R, G39R, N1161R or S1229R., optionally wherein the first amino acid modification is D37R and the second amino acid modification is selected from D1091R, N1161R, D912R, S1229R or G39R, optionally wherein the first amino acid modification is D1091R and the second amino acid modification is selected from N1161R, S1229R, and D912R.

6. The Cas9 protein according to claim 5, further comprising a third amino acid modification selected from A1160R, D1073A, D1073G, D1091G, D1091N, D1091R, D1091S, D1122R, D1206G, D1206R, D1206S, D37G, D37N, D47G, D47L, D47R, D59G, D59R, D59S, D61G, D61L, D61R, D911G, D911S, D912G, D912N, D912R, D912S, E1164R, E1226G, E1226R, E1226S, E508G, E508R, F94G, F94Y, I46A, I46R, I978R, K980R, N1161R, N501F, N501R, N501W, N501Y, Q315K, Q315R, S1229R, S566K, S566R, S57A, S57L, S57R, and T54R.

7. The Cas9 protein according to claim 6, further comprising a fourth amino acid modification selected from D1206S, D59S, D912N, D912R, D912S, E1164R, E1226G, E1226S, F94Y, I46R, Q556R, S1229R, S566R, and S57R, optionally a fifth amino acid modification selected from D1206N, D1206S, D912N, D912R, D912S, G39R, I46R, and S1229R, optionally a sixth amino acid modification selected from S1229R, S566R, and optionally a seventh amino acid modification at position D912N.

8. The Cas9 protein according to any one of the preceding claims, wherein said Cas9 protein comprises one or more mutations as defined in Table 2, or wherein said Cas9 protein comprises or consists of an amino acid sequence as defined in any one of SEQ ID NOs: 9-191, such as SEQ ID NOs: 9-144, SEQ ID NOs: 14-144, SEQ ID NOs: 30-144, SEQ ID NOs: 60-144, SEQ ID NOs: 112-144 and/or SEQ ID NOs: 140-144.

9. The Cas9 protein according to any one of the preceding claims, wherein said Cas9 protein further comprises at least one nuclear localization signal (NLS), such as VC, 53BP1, bpSV40, BRCA1_1, BRCA1 2, EWS, FUS, HIV-I _TAT, hnRNP-D, hnRNP-M, HsNPM2, HTLV-1, mpSV40, MYC, NLP, NPM2, PTHrP and/or REV_HV1H2.

10. A CRISPR-Cas system comprising:
a) a Cas9 protein comprising a polypeptide sequence having at least 95% identity, such as 97%, 98% or 99%, to SEQ ID NO: 1, wherein said polypeptide sequence comprises an amino acid modification at one or more positions relative to SEQ ID NO: 1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229, or a Cas9 protein as defined in any one of the preceding claims; and
b) a guide polynucleotide comprising a guide sequence, wherein the guide sequence is capable of hybridizing with a target sequence in a eukaryotic cell.

11. A CRISPR-Cas system comprising:
a) a nucleic acid sequence encoding a Cas9 protein comprising a polypeptide sequence having at least 95% identity, such as 97%, 98% or 99%, to SEQ ID NO: 1, wherein said polypeptide sequence comprises an amino acid modification at one or more positions relative to SEQ ID NO: 1: 37, 39, 46, 47, 54, 57, 59, 61, 94, 315, 501, 508, 556, 566, 911, 912, 978, 980, 1073, 1091, 1122, 1160, 1161, 1164, 1206, 1226 and/or 1229, or a Cas9 protein as defined in any one of claims 1-9 ; and
b) a nucleic acid sequence encoding a guide polynucleotide that comprises a guide sequence, wherein the guide sequence is capable of hybridizing with a target sequence in a eukaryotic cell.

12. The CRISPR-Cas system according to claim 10 or 11, wherein the guide polynucleotide comprises or consists of a polynucleotide sequence as defined in any one of SEQ ID NO: 192-206.

13. A eukaryotic cell or a delivery particle comprising the Cas9 protein as defined in any one of claims 1 to 9 or the CRISPR-Cas system as defined in any one of claims 10-12.

14. A method for providing site-specific modification of a target sequence in a eukaryotic cell, the method comprising:
a) introducing into the eukaryotic cell a nucleotide sequence encoding a guide polynucleotide that forms a complex with a Cas9 protein, wherein said guide polynucleotide comprises a guide sequence,
wherein the guide sequence is capable of hybridizing with a target sequence in host polynucleotide; and wherein the eukaryotic cell comprises the Cas9 protein as defined in any one of claims 1-9,
b) generating cohesive ends in the host polynucleotide with the Cas9 protein and the guide polynucleotide; and
c) ligating
i) the cohesive ends of (b) together, or
ii) a 3' end of a polynucleotide sequence of interest to a first cohesive end of the host polynucleotide, and a 5' end of the polynucleotide sequence of interest to a second cohesive end of the host polynucleotide; thereby modifying the target sequence.

15. The method according to claim 14, wherein step a) further comprises i) introducing into the eukaryotic cell a nucleotide sequence encoding the Cas9 protein as defined in any one of claims 1-9, and ii) expressing the Cas9 protein by the eukaryotic cell.
